Europäisches Patentamt

(19) European Patent Office

Office européen des brevets

(11) Publication number: **0 183 396**
**A1**

## (12) EUROPEAN PATENT APPLICATION

(21) Application number: 85307882.2

(22) Date of filing: 31.10.85

(51) Int. Cl.⁴: **A 61 M 25/00**

(30) Priority: 08.11.84 US 669695

(43) Date of publication of application:
04.06.86 Bulletin 86/23

(84) Designated Contracting States:
AT BE CH DE FR GB IT LI LU NL SE

(71) Applicant: BAXTER TRAVENOL LABORATORIES, INC.
One Baxter Parkway
Deerfield, IL 60015(US)

(72) Inventor: Belloti, Marc
1215 Willow Road Winnetka
Illinois 60093(US)

(72) Inventor: Taylor, Larry C.
5421 Shore Drive McHenry
Illinois 60050(US)

(74) Representative: MacGregor, Gordon et al,
ERIC POTTER & CLARKSON 14 Oxford Street
Nottingham, NG1 5BP(GB)

(54) Connection site protector with tapered bore.

(57) A protector (30) for a flanged connector which comprises: a pair of hollow shell halves (32,34) connected together by longitudinal hinge means, (35) said shell halves being capable of pivoting about said hinge means (35) to a closed position to form a tubular member. Latch means (44–46) are provided for releasably retaining the shell halves (32,34) in closed position. The shell halves (32,34) are substantially filled with antiseptic-carrying porous material (48) which define a central bore (50) extending from end to end of the hollow shell halves when in the closed position. In accordance with this invention, the bore (50) of the porous member is transversely larger at one end of the protector than at the other end thereof. This improves the operation of the device.

FIG.1

# CONNECTION SITE PROTECTOR WITH TAPERED BORE

## Technical Field and Prior Art

In U.S. Patent Application Serial No. 605,450 filed April 30, 1984 by William R. Knab and entitled Connection Site Protector, a protector for a flanged connector is provided. The protector comprises hollow shell halves hingedly connected together and openable and closable in a "clamshell" manner. The protector is substantially filled with antiseptic-carrying porous material. The separate pieces of porous material reside in each of the shell halves and when in the closed position define a central bore extending through the protector. The protector may be clamped onto a flange of a connector, with the bore of antiseptic-carrying porous material presenting an open end so that another connector can pass into locked relation with the flanged connector and then be removed, as desired. Thus, an antibacterial protective member is provided which can be applied and removed from a flanged connector, yet which can also permit the connection and disconnection of another connector with the first flanged connector while the protector is in position.

Initial prototypes of the above described protector have been tested with respect to a connector system for continuous ambulatory peritoneal dialysis (CAPD) sold by Travenol Laboratories, Inc. of Deerfield, Illinois, in which the connectors are of a screw threaded variety (Travenol's System II). Both of the connectors have flanges, the flange of the first connector being used to mount the clamshell type protector on the first connector. The second flange fits into the mouth of one end of the protector to form a protective shield, while a tubular portion of the second connector enters into screw thread relation with the first connector while being surrounded by the antiseptic-carrying porous material.

It has been found, however, that while it is desirable for the porous material to define a bore which is no larger than the connecting portions of the two connectors, when one does that, often the flexible porous material is caught between the two connectors as they are brought together. One must then either withdraw the two protectors and try again, or the porous material will be ripped and torn, forming particulate matter that can get into the flow path and may find its way into the peritoneal cavity of the patient who is undergoing CAPD.

In accordance with this invention, an improvement is provided in a protector system using characteristics of the protector system of the previously cited Knab patent application for improved results of operation.

Another clamshell type protector is disclosed in Dennehey et al. U.S. Patent No. 4,340,052.

Rogers Patent No. 4,354,490 discloses another type of protector for an ambulatory dialysis system. The protector may be a cylindrical member which is filled with absorbent, and which does not break apart in hinged manner as a clamshell. The absorbent may be a packed material saturated with a disinfectant. A portion of a mating connector member penetrates through an aperture, passing through the disinfectant material within the cylindrical member to enter into connection. An interlocking sleeve connection may be used between the pair of connectors, or the cylindrical member may be a separate member, open at both ends, for receiving a pair of tubular connectors.

It is contemplated that the devices as described above can be improved by the invention of this application.

## Description of the Invention

In this invention, a protector is provided for a flange connector, which comprises: a pair of hollow shell halves connected together by longitudinal hinge means. The shell halves are capable of pivoting about the hinge means to a closed position to form a tubular member. Latch means are provided for releasably retaining the shell halves in closed position. Each of the shell halves are substantially filled with antiseptic-carrying porous material and define, in the closed position, a bore extending from end to end of the protector through the porous material.

In accordance with this invention, the bore of said porous material is transversely larger at one end of the protector than at its other end, typically smoothly tapering between the two ends. Means are also provided for retaining of the protector on a flange of the connector, with the transversely larger bore typically facing outwardly relative to the end of the protector. Accordingly, a second connector may be brought into engagement with the first connector within the protector, where the enlarged outer bore end of the porous material does not initially engage the connector, but as the connector is advanced into the protector it enters into engagement with the reduced diameter bore portion to compress the porous material and cause release of the antiseptic.

The bore may be defined by separate grooves or channels in separate portions of the porous material carried by each shell half. The end portion of each groove which is of the lesser transverse dimension may be of a V shaped cross section, while the larger end of the groove may be semicircular.

Accordingly, as a second connector is placed into engagement with the first connector upon which the protector of this invention is carried, it cannot pinch portions of the porous material between the two engaging connectors, because of the transversely enlarged bore of the porous material which the second connector first encounters. However, as the connector continues to advance, it encounters a bore of diminished transverse dimension so that the second connector can compress the porous material, squeezing out the antiseptic so that antiseptic can enter into intimate contact with the two connectors.

## Description of the Drawings

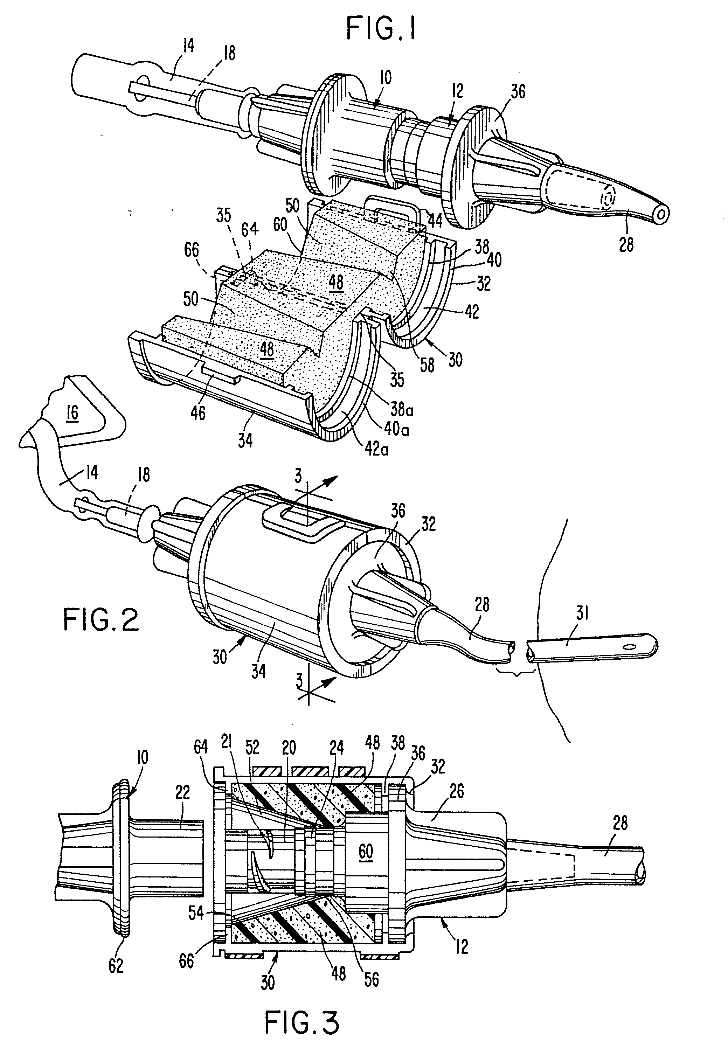

In the drawings, Figure 1 is a perspective view of a pair of connectors, one, for example, connecting with a peritoneal dialysis solution bag, and the other connecting with tubing which communicates with the peritoneal cavity of a patient. An open protector member in accordance with this invention is shown prior to attachment to one of the connectors.

Figure 2 is a perspective view, shown partially schematically, in which the protector member of Figure 1 is shown in locked position on the connectors.

Figure 3 is a sectional view taken along line 3-3 of Figure 2, with the connectors in separated relation.

## Description of Specific Embodiment

Referring to the drawings, connectors 10, 12, are shown to be of the threaded luer lock connector type, similar to the connectors of U.S. Patent No. 4,294,250 in locked relationship with each other.

Connector 10 may communicate by flexible tube 14 to a peritoneal dialysis solution bag 16. Internal frangible member 18 closes flow through tube 14 and connector 10 until broken. Frangible member 18 may be of the design shown in U.S. Patent No. 4,340,049, or any other openable seal device may be used as desired.

Connector 12 may carry a tubular projecting member 20 with screw threads 21 which fit telescopically into tubular member 22 of connector 10, and mate with internal screw threads positioned on the inner wall thereof. If desired, the interior design of connector 10 within sleeve 22 may be similar to that shown in Dennehey et al. U.S. Patent No. 4,346,703. An inner projecting member within sleeve 22 passes into the bore of tubular projecting member 20 to form a luer-type sealing connection, while sleeve 22 forms a second seal against annular land 24. Projecting member 20 and annular land 24 may be made of plastic, stainless steel, or titanium as may be desired, for example, while rear portion 26 of connector 12 may be made of molded plastic. Connector 12 may also be made of molded plastic, as well as connector 10.

As shown, connector 12 may communicate at its rear end with flexible tubing 28, which in turn may ultimately communicate with a peritoneal catheter 31, by means of another conventional connection.

Protector member 30 may comprise a pair of molded plastic hollow shell halves 32, 34, which are held together by plastic hinge 35 to permit the shell halves to assume an open position as shown in Figure 1, or a closed position as in Figure 2, in which shell halves 32, 34 are receiving and holding a flange 36 of connector 12.

As shown in Figure 1, each of the shell halves 32, 34 of protector member 30 define a pair of spaced, laterally extending ribs 38, 40 in the case of shell half 32, and 38a, 40a in the case of shell half 34. These spaced ribs respectively define grooves, 42, 42a between them which serve as a receiving slot for flange 36, where protector member 30 is closed about connector 12. Latch member 44 may close about stud 46 for releasable latching of the two shell halves together.

Each shell half 32, 34 carries on its inner surface a piece of sponge 48 or the like, which in turn may be soaked with an antiseptic such as povidone iodine. Each portion of sponge may be welded or otherwise adhered to its shell half 32, 34. Accordingly, as shown in Figure 3, sponge portions 48 may surround and protect the enclosed projecting member 20 when connector 12 is enclosed with protector member 30.

The respective of sponge portions 48 which fit in each of shell halves 32, 34, define a groove or channel 50, which forms bore 52 when the shell halves are placed in their closed position as shown in Figure 3.

Bore 52 is shown to be transversely larger at one end 54 than at the other end 56, which is, of course, a logical consequence of the shapes of grooves 50 as shown in Figure 1. Specifically, one end of groove 50 is shown to be of V shaped configuration and much smaller in transverse dimension than the other end, which is semicircular in configuration.

Accordingly, when cylindrical sleeve 22 of connector 10 is inserted into locking relation with connector 12, with protector 30 locked in place on flange 36, it passes first through the enlarged bore portion at end 54 of the protector. Thus, there is little chance of the material of sponge 48 being caught between the two connectors as they enter into telescoping relation.

As tubular portion of connector 22 continues to advance, it enters into contact with sponge 48, providing further compression. It can be seen from Figure 3 that the narrowed end 56 of bore 52 is already compressed by portion 60 of connector 12. The effect of this compression is to cause antiseptic liquid to be expelled from sponge 48 to bathe external portions of connector 12 positioned within protector 30 in the antiseptic. Further antiseptic is typically released as connector 10 is brought into locked position with connector 12 for added antibacterial effect within and adjacent to the newly formed connection between the two connectors 10, 12.

Flange 62 of connector 10 can meet flange portions 64, 66 of protector 30 to provide a closure for the system. Flange portions 64, 66, which are each semicircular and positioned within the separate shell halves 32, 34, and also serve to retain liquid antiseptic ir the system.

Accordingly, by this invention, a protector is provided which permits repeated connection and disconnection between two connectors, while causing release of antiseptic such as povidone iodine upon compression of sponge material inside the protector. The problem of tearing of sponge material and wedging of such material between the two locked connectors, interfering with good locking and the like, is substantially eliminated by the device of this invention.

The above has been offered for illustrative purposes only, and is not intended to limit the scope of the invention of this application, which is as defined in the claims below.

THAT WHICH IS CLAIMED IS:

1. In a protector for a flanged connector, which comprises: a pair of hollow shell halves connected together by longitudinal. hinge means, said shell halves being capable of pivoting about said hinge means to a closed position to form a tubular member, and a latch means for releasably retaining said shell halves in closed position, said shell halves being substantially filled with antiseptic-carrying flexible porous material defining a central bore extending therethrough when the protector is in closed position, the improvement comprising, in combination: the bore of said porous material being transversely larger at one end of the protector than at the other end thereof whereby a second connector can project into the enlarged bore end of said protector closed about a flanged connector without engaging the porous material, and said second connector upon projecting farther into said protector can compress portions of said porous material to release antiseptic agent.

2. The protector of Claim 1 in which a pair of spaced, laterally extending ribs are positioned in the inner surface of at least one shell half adjacent the other end thereof to receive and hold a flange of said flanged connector between them, whereby said shell halves may be closed about the flange of said connector to be releasably retained on said connector.

3. The protector of claim 1 in which the porous material of each shell half defines a groove which is V shaped at its narrower end and semicircular at its larger end, said grooves joining together in registry with each other to form said bore when the protector is closed.

4. The protector of Claim 1 in which laterally extending ribs are positioned on the inner surfaces of said shell halves adjacent said one end thereof to retain said porous material and prevent it from being dislodged as a second connector is being withdrawn from locked engagement with the flanged connector within said protector.

5. In a protector for a flanged connector which comprises: a pair of hollow shell halves connected together by longitudinal hinge means, said shell halves being capable of pivoting about said hinge means to a closed position to form a tubular member, and a latch means for releasably retaining said shell halves in closed position, said shell halves being substantially filled with antiseptic-carrying flexible porous material defining a central bore extending therethrough when the protector is in closed position, the improvement comprising, in combination: the bore of said porous material being transversely larger at one end of the protector than at the other end thereof, whereby a second connector can project into the enlarged bore end of said protector closed about a flanged connector without engaging the porous material, and said second connector upon projecting further into said protector can compress portions of said porous material to release antiseptic agent, a pair of spaced, laterally extending ribs being positioned in the inner surface of said shell halves adjacent the other end thereof to receive and hold a flange of said flanged connector between them, whereby said shell halves may be closed about the flange of said connector to be releasably retained on said connector, and in which laterally extending ribs are positioned on the inner surfaces of said shell halves adjacent to said one end thereof to retain said porous material and prevent it from being dislodged as a second connector is being withdrawn from locked engagement with the flanged connector within said protector.

6. The protector of claim 5 in which the porous material of each shell half defines a groove which is V shaped at its narrower end and semicircular at its larger end, said grooves joining together in registry with each other to form said bore when the protector is closed.

0183396

FIG.1

FIG.2

FIG.3

European Patent
Office

**EUROPEAN SEARCH REPORT**

0183396
Application number

EP 85 30 7882

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | EP-A-0 073 432 (GROSS)<br>* Page 16, lines 6-21; figures 2,16 * | 1-6 | A 61 M 25/00 |
| Y,D | US-A-4 354 490 (ROGERS)<br>* Column 3, lines 29-33; figure 3 * | 1-6 | |
| A,D | US-A-4 340 052 (DENNEHEY et al.)<br>* Abstract; figure 4 * | 1 | |
| E,D | WO-A-8 505 040 (KNAB)<br>* Abstract; figure 3 * | 1 | |
| | ----- | | |

TECHNICAL FIELDS SEARCHED (Int. Cl.4)

A 61 M

The present search report has been drawn up for all claims

| Place of search | Date of completion of the search | Examiner |
|---|---|---|
| THE HAGUE | 12-02-1986 | EHRSAM F.J.A. |